# EUROPÄISCHE PATENTSCHRIFT

(11) **EP 3 307 232 B1**
(45) Veröffentlichungstag und Bekanntmachung des Hinweises auf die Patenterteilung: **19.02.2020**
(21) Anmeldenummer: 16720082.3
(22) Anmeldetag: 25.04.2016
(51) Int. Cl.: A61K 8/37, A61Q 5/06, A61Q 5/10, A61Q 15/00, A61K 8/04

(54) **VERWENDUNG VON ZITRONENSÄUREESTERN ALS KORROSIONSINHIBITOR IN ALKOHOLISCHEN, ALS AEROSOL VERSPRÜHBAREN KOSMETISCHEN ZUSAMMENSETZUNGEN**
USE OF CITRIC ACID ESTERS AS CORROSION INHIBITORS IN ALCOHOLIC COSMETIC COMPOSITIONS SPRAYABLE AS AEROSOLS
UTILISATION D'ESTERS D'ACIDE CITRIQUE EN TANT QU'INHIBITEURS DE CORROSION DANS DES COMPOSITIONS COSMÉTIQUES ALCOOLIQUES PULVÉRISABLES UTILISÉES SOUS FORME D'AÉROSOL

(30) Priorität: 09.06.2015 DE 102015210481
(43) Veröffentlichungstag der Anmeldung: 18.04.2018
(73) Patentinhaber: Henkel AG & Co. KGaA, 40589 Düsseldorf (DE)
(72) Erfinder: DÖRING, Thomas, 41540 Dormagen (DE); TECKENBROCK, Gertraud, 45549 Sprockhövel (DE)
(86) Internationale Anmeldenummer: PCT/EP2016/059190
(87) Internationale Veröffentlichungsnummer: WO 2016/198202

(56) Entgegenhaltungen:
- EP-A1- 0 200 001
- CN-A- 101 750 911
- DE-A1-102011 084 155
- DE-A1-102011 088 967

## Beschreibung

Die vorliegende Erfindung betrifft die Verwendung von Zitronensäureestern als Korrosionsinhibitoren in alkoholischen kosmetischen Zusammensetzungen, welche mindestens einen Elektrolyten, 0,02 bis 2,0 Gew.-% Wasser sowie mindestens ein Treibmittel enthalten.

Treibmittelhaltige Zusammensetzungen werden in sogenannten Aerosolcontainern (auch als Sprühdosen bezeichnet) abgefüllt. Aus diesen Containern werden die darin befindlichen Zusammensetzungen mit Hilfe eines Treibmittels als Aerosole versprüht. Die Verwendung von Aerosolcontainern zur Versprühung von kosmetischen Mitteln, wie Deodorantien, Antitranspirantien, Haarfärbemitteln, Haarsprays und Haarstylingmitteln, bietet zahlreiche Vorteile. So wird zum einen die Zusammensetzung auf der Anwendungsoberfläche gleichmäßig verteilt, zum anderen lässt sich diese einfach und gut kontrollierbar dosieren. Weiterhin kann der Verbrauch der Zusammensetzung pro Anwendung verringert werden, so dass Verpackungsabfall infolge längerer Verwendbarkeit vermindert wird.

Als druckfeste Behälter für derartige Aerosolcontainer werden zumeist Gefäße aus Metall (Aluminium, Weißblech, Zinn), geschütztem bzw. nicht-splitterndem Kunststoff oder aus Glas, welcher außen mit Kunststoff beschichtet ist, eingesetzt. Bei Verwendung von Behältern aus Kunststoff, Glas oder Aluminium treten keine Beschädigungen der Behälter durch Korrosion auf, jedoch sind diese Behälter in ihrer Herstellung deutlich teurer als üblicherweise verwendete Behälter aus Weißblech oder Zinn. Diese Behälter werden daher nur in sehr geringer Menge oder überhaupt nicht eingesetzt.

Wegen der guten kommerziellen Verfügbarkeit und günstigen Herstellkosten werden als Aerosolbehälter zumeist Behälter aus Stahl und Zinn eingesetzt. Bei diesen Behältern tritt jedoch durch bestimmte Inhaltsstoffe der enthaltenen kosmetischen Mittel unerwünschte Korrosion auf.

Die in diesen Aerosolcontainern auftretende Korrosion läuft normalerweise elektrochemisch ab, wobei Elektronen von dem Metall des Behälters und/oder Ventiltellers auf Elektronenakzeptoren in dem kosmetischen Mittel übertragen werden. Das Metall des Behälters wird hierbei oxidiert und auf diese Weise zerstört. Insbesondere der Einsatz von Elektrolyten im Zusammenhang mit wässrigen Lösungen fördert die Korrosion von metallischen Aerosolbehältern, da Elektrolyte in wässrigen Lösungen in Ionen dissoziieren und so eine elektrische Leitfähigkeit hervorrufen, welche die elektrochemische Korrosion weiter begünstigt. Die Korrosion dieser Behälter und/oder Ventilteller führt nicht nur zu einer Verunreinigung der enthaltenen kosmetischen Mittel, sondern auch zur Zerstörung des Aerosolbehälters und/oder Ventiltellers durch Lochbildung oder Risse und somit zu einer verminderten Lagerstabilität befüllter Aerosolcontainer.

Zur Vermeidung der Korrosion dieser Behälter werden im Stand der Technik zum einen metallische Aerosolcontainer verwendet, deren Teile an den mit dem kosmetischen Mitteln in Kontakt stehenden Stellen mit Schutzlacken beschichtet sind. Derartige Schutzlacke sind jedoch aufwendig zu applizieren und verteuern daher den Herstellprozess der Aerosolgefäße und Ventile erheblich. Zum anderen werden die eingesetzten Metalle, insbesondere Stahlbleche, mit einer dünnen Zinnbeschichtung versehen, welche eine höhere Korrosionsbarriere gegen die in den kosmetischen Mitteln enthaltenen korrosiven Stoffe, insbesondere Chloridionen, Säuren und Basen, aufweist als das unbehandelte Stahlblech. Allerdings besteht die Gefahr, dass die Verzinnung nicht absolut gleichmäßig appliziert ist. In diesem Fall kann es an Fehlstellen zu sogenannter Lochkorrosion kommen.

Die Korrosion von Behältern aus Zinn und Stahl kann weiterhin vermeiden bzw. vermindern werden, wenn kosmetische Mittel eingesetzt werden, welche wasserfrei sind bzw. ausschließlich wasserfreie flüchtige organische Lösungsmittel enthalten. Jedoch haben Gesetzesänderungen und Kundenwünsche dazu geführt, dass die Menge an flüchtigen organischen Lösungsmitteln (auch als sogenannte volatile organic compounds oder VOC bezeichnet) reduziert und durch Wasser und/oder Alkohole, welche üblicherweise Restmengen an Wasser enthalten, ersetzt werden muss. Der Einsatz von Wasser oder wasserhaltigen Alkoholen führt jedoch zu einer Erhöhung der Korrosionsgefahr metallischer Aerosolcontainer.

Daher werden bei Verwendung von wasserhaltigen und als Aerosol versprühbaren kosmetischen Mitteln im Stand der Technik häufig Korrosionsinhibitoren eingesetzt. Derartige Inhibitoren können als Beschichtung auf die mit der kosmetischen Zusammensetzung in Kontakt kommenden Teile des Aerosolbehälters aufgebracht oder den kosmetischen Zusammensetzungen zugesetzt werden.

Im Stand der Technik bekannte Korrosionsinhibitoren sind beispielsweise Alkalimetallnitrite und - benzoate, Borate, Alkanolamine und -amide, Aminverbindungen wie Morpholin, Amide oder auch Silicone. Ein Nachteil der verwendeten Nitrite und Borate ist deren reizende Wirkung, weshalb sie als Korrosionsinhibitor für kosmetische Mittel, welche mit der menschlichen Haut in Berührung kommen, nur bedingt geeignet sind. Die bekannten Amine und Amide besitzen zwar eine ausreichende kosmetische Verträglichkeit, weisen jedoch eine unzureichende korrosionsinhibierende Wirkung auf, so dass die Korrosion der Aerosolbehälter langfristig nicht zufriedenstellend verhindert wird.

Es besteht somit weiterhin ein Bedarf an Korrosionsinhibitoren, welche eine hohe korrosionsinhibierende Wirkung aufweisen, keine negativen Einflüsse auf die chemischen oder ästhetischen Eigenschaften der kosmetischen Zusammensetzungen aufweisen und kosmetische sowie toxikologisch unbedenklich sind. Der vorliegenden Erfindung lag die Aufgabe zugrunde, einen Korrosionsinhibitor bereitzustellen, welcher eine hohe korrosionsinhibierende Wirkung aufweist und sich daher insbesondere für den Einsatz in elektrolythaltigen wässrigen kosmetischen Mitteln, welche als Aerosol versprüht werden sollen, eignet. Weiterhin soll dieser Korrosionsinhibitor kosmetisch und toxikologisch gut verträglich sein und keine negativen Wechselwirkungen mit den üblichen Inhaltsstoffen der kosmetischen Mittel aufweisen.

Es wurde nun überraschend gefunden, dass die Verwendung von Estern der Zitronensäure in alkoholischen kosmetischen Zusammensetzungen, welche Wasser sowie mindestens einen Elektrolyten enthalten und als Aerosol versprüht werden sollen, zu einer hervorragenden korrosionsinhibierende Wirkung führt. Weiterhin sind derartige Ester kosmetisch und toxikologisch unbedenklich und führen nicht zu negativen Wechselwirkungen mit anderen Inhaltsstoffen von kosmetischen Mitteln.

Gegenstand der vorliegenden Erfindung ist somit die Verwendung eines Esters der Zitronensäure der Formel (I) 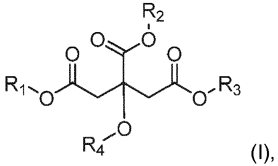 worin
R₁ bis R₃, jeweils unabhängig voneinander, für eine C₁-C₁₀-Alkylgruppe stehen, und
R₄ für Wasserstoff oder eine C₁-C₄-Alkylgruppe steht,
als Korrosionsinhibitor in alkoholischen kosmetischen Zusammensetzungen, welche mindestens ein Treibmittel, mindestens einen Elektrolyten in einer Gesamtmenge von 0,01 bis 1,0 Gew.-% sowie Wasser in einer Gesamtmenge von 0,02 bis 2,0 Gew.-%, jeweils bezogen auf das Gesamtgewicht der alkoholischen kosmetischen Zusammensetzung, enthalten.

Durch den Einsatz von bestimmten Estern der Zitronensäure können alkoholische kosmetische Zusammensetzungen formuliert werden, welche sowohl Wasser als auch Elektrolyte enthalten und als Aerosol versprühbar sind, ohne dass es zur Korrosion der Aerosolbehälter und/oder Ventilteller kommt. Weiterhin weisen diesen Ester eine hervorragende kosmetische Verträglichkeit auf und sind toxikologisch vollkommen unbedenklich. Zudem führt deren Einsatz nicht zu negativen Wechselwirkungen mit weiteren Inhaltsstoffen der kosmetischen Mittel, so dass eine Anpassung bereits bestehender Formulierungen nicht erforderlich ist.

Unter dem Begriff "Korrosionsinhibitor" werden erfindungsgemäß solche Stoffe verstanden, deren Anwesenheit in der kosmetischen Zusammensetzung die Korrosion des die Zusammensetzung umgebenden Metallbehälters und/oder Ventiltellers hemmt. Die Inhibierung der Korrosion kann hierbei durch Hemmung der elektrochemischen Reaktion sowie durch physikalische oder chemische Interaktion erfolgen. Bei der physikalischen Interaktion findet eine Absorption des Inhibitors durch elektrostatische Anziehung oder herrschende Van-der-Waals-Kräfte auf der Metalloberfläche statt, wohingegen bei der chemischen Interaktion durch eine Reaktion des Inhibitors mit dem zu schützenden Metall eine Schutzschicht gebildet wird.

Weiterhin werden unter dem Begriff "alkoholische Zusammensetzungen" im Sinne der vorliegenden Erfindung Zusammensetzungen verstanden, welche mindestens einen einwertigen C₁-C₄-Alkohol, insbesondere Ethanol, in einer Menge von mindestens 5 Gew.-%, bezogen auf das Gesamtgewicht der kosmetischen Zusammensetzung, enthalten. Der mindestens eine einwertige C₁-C₄-Alkohol wird bevorzugt als Lösungsmittel, insbesondere kosmetisch verträglicher Träger, eingesetzt.

Zudem werden unter dem Begriff "Elektrolyte" im Sinne der vorliegenden Erfindung Stoffe verstanden, welche durch Dissoziation in Lösung, insbesondere in wässriger oder wässrig-alkoholischer Lösung, Ionen freisetzen können. Durch die Freisetzung von Ionen und deren Beweglichkeit bildet die Lösung ein ionenleitendes Medium mit elektrischer Leitfähigkeit.

Darüber hinaus sind unter dem Begriff der "Fettsäure", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff des "Fettalkohols" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die Angabe Gew.-% bezieht sich vorliegend, sofern nichts anderes angegeben ist, auf das Gesamtgewicht der treibmittelhaltigen kosmetischen Zusammensetzung.

Erfindungsgemäß werden als Korrosionsinhibitoren für alkoholische kosmetische Zusammensetzungen spezielle Ester der Zitronensäure der Formel (I) eingesetzt. In dieser Formel können die Reste R₁ bis R₃ für C₁-C₁₀-Alkylgruppen stehen. Beispiele für derartige Gruppen sind Methyl-, Ethyl-, Propyl-, Isopropyl-, Hydroxpropyl, Butyl-, Sec-Butyl-, Isobutyl-, tert-Butyl-, Hydroxybutyl-, Pentyl-, Hexyl-, Heptyl-, Octyl-, Nonyl- und Decylgruppen. Der Rest R₄ steht für C₁-C₄-Alkylgruppen, beispielsweise Methyl-, Ethyl-, Propyl-, Isopropyl-, Hydroxpropyl, Butyl-, Sec-Butyl-, Isobutyl-, tert-Butyl- und Hydroxybutylgruppen.

Vorzugsweise werden im Rahmen der vorliegenden Erfindung solche Ester der Zitronensäure der Formel (I) eingesetzt, in welchen die Reste R₁ bis R₃ für Alkylgruppen mit einer bestimmten Kettenlänge stehen. Bevorzugte Verwendungen der vorliegenden Erfindung sind daher dadurch gekennzeichnet, dass in der Formel (I) die Reste R₁ bis R₃, jeweils unabhängig voneinander, für eine C₁-C₈-Alkylgruppe, vorzugsweise für eine C₁-C₆-Alkylgruppe, bevorzugt für eine C₁-C₄-Alkylgruppe, insbesondere für eine C₂-Alkylgruppe, stehen. Der Einsatz von Zitronensäureestern der Formel (I), in welcher die Reste R₁ bis R₃ jeweils für eine C₂-Alkylgruppe, also eine Ethylgruppe, stehen, hat sich als besonders vorteilhaft in Bezug auf die korrosionsinhibierende Wirkung von alkoholischen kosmetischen Mitteln, welche Wasser sowie mindestens einen Elektrolyten enthalten, erwiesen.

Erfindungsgemäß vorteilhaft werden Zitronensäureester der Formel (I) eingesetzt, in welcher der Rest R₄ für Wasserstoff steht. Bevorzugte erfindungsgemäße Verwendungen sind daher dadurch gekennzeichnet, dass in der Formel (I) der Rest R₄ für Wasserstoff steht.

Eine besonders bevorzugte Ausführungsform der vorliegenden Erfindung ist demnach dadurch gekennzeichnet, dass ein Ester der Zitronensäure der Formel (la) 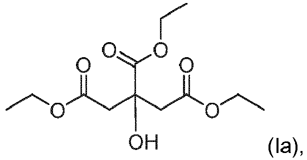 verwendet wird. Zitronensäureester der Formel (la) sind unter der INCI-Bezeichnung Triethylcitrat bekannt und weisen die CAS-Nummer 77-93-0 auf. Durch den Einsatz von Triethylcitrat in alkoholischen kosmetischen Zusammensetzungen, welche Wasser sowie mindestens einen Elektrolyten enthalten, kann die Korrosion von metallhaltigen Aerosolbehältern und/oder Ventiltellern nahezu vollständig, insbesondere vollständig, vermieden werden. Weiterhin ist Triethylcitrat kosmetisch gut verträglich und toxikologisch vollkommen unbedenklich. Darüber hinaus führt der Einsatz von Triethylcitrat nicht zu unerwünschten Wechselwirkungen mit weiteren Inhaltsstoffen, so dass es gängigen Formulierungen für treibmittelhaltige Zusammensetzungen ohne weitere Anpassung zugesetzt werden kann.

Der mindestens eine Ester der Zitronensäure der Formel (I) wird bevorzugt in bestimmten Mengenbereichen eingesetzt. Daher ist es erfindungsgemäß vorteilhaft, wenn der Ester der Zitronensäure der Formel (I) oder der Formel (la) in einer Gesamtmenge von 0,5 bis 5,0 Gew.-%, vorzugsweise von 0,5 bis 4,0 Gew.-%, bevorzugt von 0,5 bis 3,0 Gew.-%, insbesondere von 1,0 bis 2,0 Gew.-%, bezogen auf das Gesamtgewicht der alkoholischen kosmetischen Zusammensetzung, verwendet wird. Der Einsatz der zuvor angeführten Gesamtmengen des Zitronensäureesters der Formel (I) bzw. (la), führt zu einer guten Korrosionsinhibierung, ohne jedoch Inkompatibilitäten mit weiteren Inhaltsstoffen der kosmetischen Zusammensetzung zu verursachen.

Bei den im Rahmen der vorliegenden Erfindung verwendeten kosmetischen Zusammensetzungen handelt es sich um alkoholische kosmetische Zusammensetzungen. Diese Zusammensetzungen enthalten mindestens einen C₁-C₄-Alkohol. Bevorzugte Verwendungen gemäß der vorliegenden Erfindung sind daher dadurch gekennzeichnet, dass die alkoholische kosmetische Zusammensetzung mindestens einen einwertigen C₁-C₄-Alkohol, ausgewählt aus der Gruppe von Methanol, Ethanol, Propanol, iso-Propanol, Butanol sowie deren Mischungen, insbesondere Ethanol, in einer Gesamtmenge von 15 bis 30 Gew.-%, vorzugsweise 18 bis 28 Gew.-%, insbesondere 20 bis 24 Gew.-%, bezogen auf das Gesamtgewicht der alkoholischen kosmetischen Zusammensetzung, enthält.

Es ist erfindungsgemäß weiterhin bevorzugt, wenn die kosmetische Zusammensetzung Wasser in einer Gesamtmenge von 0,05 bis 1,8 Gew.-%, vorzugsweise von 0,1 bis 1,6 Gew.-%, bevorzugt von 0,2 bis 1,5 Gew.-%, insbesondere von 0,5 bis 1,5 Gew.-%, bezogen auf das Gesamtgewicht der alkoholischen kosmetischen Zusammensetzung, enthält. Bei Verwendung der Zitronensäureester der Formel (I) bzw. (la) können daher wasserhaltige alkoholische Lösungsmittel, insbesondere wasserhaltige ethanolische Lösungsmittel, in Kombination mit Elektrolyten eingesetzt werden, ohne dass eine Korrosion der metallischen Aerosolcontainer und/oder Ventilteller auftritt. Wasserhaltige alkoholische Lösungsmittel, insbesondere wasserhaltiges Ethanol, sind gegenüber wasserfreien Lösungsmitteln kostengünstiger herstellbar und weisen eine gute kommerzielle Verfügbarkeit auf.

Die kosmetischen Mittel enthalten weiterhin mindestens einen Elektrolyten. Als Elektrolyt werden bevorzugt kosmetisch verträgliche Salze, Säuren und Basen eingesetzt. Bevorzugte erfindungsgemäße Verwendungen sind daher dadurch gekennzeichnet, dass der mindestens eine Elektrolyt ausgewählt ist aus der Gruppe von anorganischen Salzen, insbesondere Alkalimetalsalzen; Salzen von di- und trivalenten Kationen sowie N-Alkylamidoalkyl-N,N-dialkyl-N-[hydroxyalkyl]ammonio]phosphaten; organischen Salzen, insbesondere N-Octyl-1-[10-(4-octyliminopyridin-1-yl)decyl]pyridin-4-iminhydrochlorid, 1-Hexadecylpyridiniumchloriden, Silbercitraten, Zink-bis[(9Z,12R)-12-hydroxy-9-octadecenoat], Kaliumcapryloylglutamaten, Benzalkoniumchloriden, Benzethoniumchloriden, 2-Butyloctansäuren, Calciummagnesiumsilikaten, Hexadecyltrimethylammoniumchloriden; anorganischen und organischen Säuren, anorganischen und organischen Basen, insbesondere Ammoniak und Aminen, sowie deren Mischungen.

In diesem Zusammenhang ist es besonders bevorzugt, wenn als Elektrolyt eine Verbindung eingesetzt wird, welche bakterienhemmende Eigenschaften aufweist und somit einer deodorierende Wirkung der kosmetischen Mittel gewährleistet. Unter deodorierender Wirkung wird hierbei die Vermeidung von unangenehmen Gerüchen, insbesondere unter der Achselhöhle, verstanden, welche durch bakterielle Zersetzungsprozesse des Schweißes, insbesondere des Achselschweißes, verursacht wird. In diesem Zusammenhang ist es daher besonders vorteilhaft, wenn der mindestens eine Elektrolyt ausgewählt ist aus der Gruppe von N-Alkylamidoalkyl-N,N-dialkyl-N-[hydroxyalkyl]ammonio]phosphaten der Formel (II) 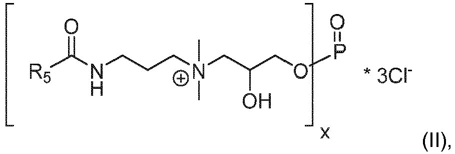 worin
R₅ für einen C₈-C₁₈-Alkylrest, insbesondere einen Cocoylrest, steht, N-Octyl-1-[10-(4-octyliminopyridin-1-yl)decyl]pyridin-4-iminhydrochlorid, 1-Hexadecyl-pyridiniumchloriden, Silbercitraten, Zink-bis[(9Z,12R)-12-hydroxy-9-octadecenoat] sowie deren Mischungen. Der Rest R₅ in der Formel (II) kann bevorzugt für einen Cocoylrest stehen. Hierunter wird eine Mischung von C₈-C₁₈-Carbonsäureresten verstanden, d. h. die Formel (II) liegt als uneinheitliche Mischung mit verschiedenen Carbonsäureresten mit 8 bis 18 Kohlenstoffatomen vor. Verbindungen der Formel (II) sind unter der INCI-Bezeichnung Cocamidopropyl PG-Dimonium Chlorid Phosphat bekannt und beispielsweise unter dem Handelsnamen Arlasilk PTC von der Firma Croda kommerziell erhältlich.

Die Verbindung N-Octyl-1-[10-(4-octyliminopyridin-1-yl)decyl]pyridin-4-iminhydrochlorid wird in der INCI-Nomenklatur auch als Octenidine HCl bezeichnet und weist die CAS-Nummer 70775-75-6 auf. Die Verbindung 1-Hexadecyl-pyridiniumchloriden mit der CAS-Nummer 123-03-5 wird gemäß der INCI-Nomenklatur auch als Cetylpyridinium Chloride bezeichnet. Unter der Bezeichnung Zink-bis[(9Z,12R)-12-hydroxy-9-octadecenoat] ist nach der INCI-Nomenklatur Zinc Ricinoleate mit der CAS-Nummer 13040-19-2 zu verstehen. Die zuvor angeführten Verbindungen dissoziieren in Lösung, insbesondere in alkoholischer wässriger Lösung, in Ionen und können daher in wasserhaltigen Lösungen die Korrosion von metallischen Aerosolbehältern und/oder Ventiltellern beschleunigen bzw. begünstigen. Bei erfindungsgemäßer Verwendung von Zitronensäureestern der Formel (I) bzw. (la) können diese Stoffe jedoch ohne Korrosionsgefahr in alkoholischen wasserhaltigen Lösungen, insbesondere ethanolischen wasserhaltigen Lösungen, eingesetzt werden. Auf diese Weise lassen sich als Aerosol versprühbare alkoholische wasserhaltige Lösungen formulieren, welche eine hervorragende Deodorantwirkung aufweisen und nur einen geringen Anteil an bzw. keine flüchtigen organischen Lösungsmittel (VOC) enthalten.

Der mindestens eine Elektrolyt wird vorteilhafterweise in bestimmten Mengenbereichen eingesetzt. Erfindungsgemäß bevorzugte Verwendungen sind daher dadurch gekennzeichnet, dass die kosmetische Zusammensetzung den mindestens einen Elektrolyten in einer Gesamtmenge von 0,01 bis 0,3 Gew.-%, vorzugsweise von 0,02 bis 0,2 Gew.-%, insbesondere von 0,03 bis 0,1 Gew.-%, bezogen auf das Gesamtgewicht der alkoholischen kosmetischen Zusammensetzung, enthält. Der Einsatz der zuvor angeführten Gesamtmengen des Elektrolyts, insbesondere eines deodorierend wirkenden Elektrolyts, stellt eine ausreichende deodorierende Wirkung durch Hemmung von Bakterien, welche den Schweiß zu unangenehm riechenden Verbindungen zersetzen, sicher.

Bei den erfindungsgemäß verwendeten kosmetischen Zusammensetzungen handelt es sich um als Aerosol versprühbare Zusammensetzungen. Solche Zusammensetzungen enthalten mindestens ein Treibmittel. Bevorzugt ist das mindestens eine Treibmittel ausgewählt aus der Gruppe von Propan, Propen, Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene sowie deren Mischungen, insbesondere Propan und/oder Butan. Unter Butan wird erfindungsgemäß n-Butan, iso-Butan und Gemische aus n-Butan und iso-Butan verstanden.

Das mindestens eine Treibmittel wird vorteilhafterweise in bestimmten Gesamtmengen eingesetzt, um eine ausreichende Versprühbarkeit und Größenverteilung der kosmetischen Zusammensetzung zu erreichen. Es ist daher im Rahmen der vorliegenden Erfindung bevorzugt, wenn die kosmetische Zusammensetzung das mindestens eine Treibmittel in einer Gesamtmenge von 50 bis 95 Gew.%, vorzugsweise 60 bis 85 Gew.%, insbesondere 70 bis 80 Gew.-%, bezogen auf das Gesamtgewicht der alkoholischen kosmetischen Zusammensetzung, enthält.

Eine besonders bevorzugte Ausführungsform der vorliegenden Erfindung ist daher dadurch gekennzeichnet, dass mindestens ein Zitronensäureester der Formel (la) 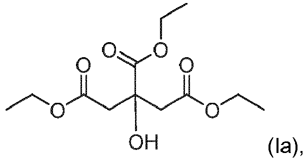 als Korrosionsinhibitor in alkoholischen kosmetischen Zusammensetzungen, welche Propan und/oder Butan in einer Gesamtmenge von 70 bis 80 Gew.-%, mindestens einen Elektrolyten in einer Gesamtmenge von 0,03 bis 0,1 Gew.-% sowie Wasser in einer Gesamtmenge von 0,5 bis 1,5 Gew.-%, jeweils bezogen auf das Gesamtgewicht der alkoholischen kosmetischen Zusammensetzung, enthalten, verwendet wird,
wobei der mindestens eine Elektrolyt ausgewählt ist aus der Gruppe von N-Alkylamidoalkyl-N,N-dialkyl-N-[hydroxyalkyl]ammonio]phosphaten der Formel (II) 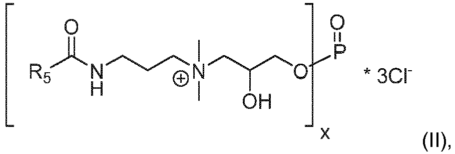 worin
R₅ für einen C₈-C₁₈-Alkylrest, insbesondere einen Cocoylrest, steht, N-Octyl-1-[10-(4-octyliminopyridin-1-yl)decyl]pyridin-4-iminhydrochlorid, 1-Hexadecyl-pyridiniumchloriden, Silbercitraten, Zink-bis[(9Z,12R)-12-hydroxy-9-octadecenoat] sowie deren Mischungen und
wobei der Zitronensäureester der Formel (la) in einer Gesamtmenge von 1,0 bis 2,0 Gew.-%, bezogen auf das Gesamtgewicht der alkoholischen kosmetischen Zusammensetzung, verwendet wird.

Durch die Verwendung des Zitronensäureesters der Formel (la) können alkoholische wässrige kosmetische Zusammensetzung mit Elektrolyten, formuliert werden, welche sich aus metallhaltigen Aerosolbehältern als Aerosole versprühen lassen, ohne dass es durch die Anwesenheit von Wasser und Elektrolyten zu einer Korrosion der metallhaltigen Aerosolbehälter durch die alkoholische kosmetische Zusammensetzung kommt. Da die zuvor genannten Elektrolyte eine effektive deodorierende Wirkung aufweisen, ist auf diese Weise die Formulierung von wasserhaltigen und aus metallhaltigen Containern als Aerosol versprühbaren Deodorantzusammensetzung möglich.

Es kann im Rahmen der vorliegenden Erfindung weiterhin vorgesehen sein, dass die alkoholische kosmetische Zusammensetzung ein von Wasser und dem einwertigen C₁-C₄-Alkohol verschiedenes Lösungsmittel enthält. Bevorzugte erfindungsgemäße Verwendungen sind daher dadurch gekennzeichnet, dass die alkoholische kosmetische Zusammensetzung zusätzlich ein von Wasser verschiedenes Lösungsmittel, ausgewählt aus der Gruppe von Ethylenglykol, 1,2-Propylenglykol, 1,3-Propylenglykol, Glyzerin, n-Butanol, 1,3-Butylenglykol sowie deren Mischungen in einer Gesamtmenge von 15 bis 30 Gew.-%, vorzugsweise 18 bis 28 Gew.-%, insbesondere 20 bis 24 Gew.-%, bezogen auf das Gesamtgewicht der alkoholischen kosmetischen Zusammensetzung, enthält.

Um die deodorierende Wirkung weitergehend zu verstärken, kann es erfindungsgemäß vorteilhaft sein, zusätzlich zu dem mindestens einen Elektrolyten mit deodorierender Wirkung einen weiteren Deodorantwirkstoff einzusetzen. Es ist daher erfindungsgemäß bevorzugt, wenn die alkoholischen kosmetische Zusammensetzung zusätzlich einen Deodorantwirkstoff, ausgewählt aus der Gruppe von Silbersalzen, aromatischen Alkoholen, insbesondere 2-Benzylheptan-1-ol sowie Mischungen von 2-Benzylheptan-1-ol und Phenoxyethanol, 1,2-Alkandiolen mit 5 bis 12 Kohlenstoffatomen, insbesondere 3-(2-Ethylhexyloxy)-1,2-propandiol, Wirkstoffen gegen Exoesterasen, insbesondere gegen Arylsulfatase, Lipase, beta-Glucuronidase und Cystathion-β-lyase, Geruchsabsorbern, insbesondere Silicate, wie Montmorillonit, Kaolinit, llit, Beidellit, Nontronit, Saponit, Hectorit, Bentonit, Smectit und Talkum, Zeolithe, Zinkricinoleat, Cyclodextrine, desodorierend wirkenden Ionenaustauschern, keimhemmenden Mitteln, präbiotisch wirksamen Komponenten sowie deren Mischungen, in einer Gesamtmenge von 0,0001 bis 10 Gew.-%, vorzugsweise von 0,001 bis 5,0 Gew.-%, bevorzugt von 0,01 bis 2,5 Gew.-%, insbesondere von 0,1 bis 1,0 Gew.-%, bezogen auf das Gesamtgewicht der alkoholischen kosmetischen Zusammensetzung, enthält.

Die kosmetische Zusammensetzung kann weiterhin mindestens ein Konservierungsmittel enthalten, um die Haltbarkeit und damit die Anwendbarkeit der alkoholischen kosmetischen Zusammensetzung zu verlängern. Es kann daher bevorzugt sein, wenn die alkoholischen kosmetische Zusammensetzung zusätzlich ein Konservierungsmittel, ausgewählt aus der Gruppe von Iodopropinylbutylcarbamaten, Parabenen, Phenoxyethanol, Benzoesäure und deren Salzen, Dibromdicyanobutan, 2-Brom-2-nitropropan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate, 2-Benzylheptanol, 1,2-Hexandiol sowie deren Mischungen, in einer Gesamtmenge von 0,0001 bis 1,0 Gew.-%, vorzugsweise von 0,001 bis 0,8 Gew.-%, bevorzugt von 0,01 bis 0,5 Gew.-%, insbesondere von 0,05 bis 0,3 Gew.-%, bezogen auf das Gesamtgewicht der alkoholischen kosmetischen Zusammensetzung, enthält.

Besonders bevorzugt werden im Rahmen der vorliegenden Erfindung Elektrolyte eingesetzt, welche gleichzeitig deodorierende Eigenschaften aufweisen. Daher handelt es sich bei der alkoholischen kosmetischen Zusammensetzung vorteilhafterweise um eine Deodorantzusammensetzung, welche unter Einsatz von Treibmittel aus Aerosolcontainern, vorzugsweise metallhaltigen Aerosolcontainern, als Aerosol versprühbar ist. Besonders bevorzugte erfindungsgemäße Verwendungen sind daher dadurch gekennzeichnet, dass die alkoholische kosmetische Zusammensetzung eine Deodorantzusammensetzung ist, welche als Aerosol versprühbar ist. Unter Aerosol wird hierbei eine Dispersion von flüssigen Tröpfchen der alkoholischen kosmetischen Zusammensetzung in dem gasförmigen Treibmittel verstanden.

In nachfolgenden Tabellen sind besonders bevorzugte Ausführungsformen der erfindungsgemäßen Verwendung aufgeführt (alle Angaben in Gew.-%, sondern nichts anderes angegeben ist). Hierbei handelt es sich um Deodorantzusammensetzungen, welche als Aerosole versprühbar sind.

| Nr. | Verwendeter Ester der Zitronensäure | Menge des verwendeten Esters der Zitronensäure |
|---|---|---|
| E1 | Ester der Formel (I) | 0,5 bis 5,0 |
| E2 | Ester der Formel (I) | 0,5 bis 4,0 |
| E3 | Ester der Formel (I) | 0,5 bis 3,0 |
| E4 | Ester der Formel (I) | 1,0 bis 2,0 |
| E5 | Ester der Formel (Ia) | 0,5 bis 5,0 |
| E6 | Ester der Formel (Ia) | 0,5 bis 4,0 |
| E7 | Ester der Formel (Ia) | 0,5 bis 3,0 |
| E8 | Ester der Formel (Ia) | 1,0 bis 2,0 |

In den nachfolgenden alkoholischen kosmetischen Zusammensetzungen K1 bis K60 werden jeweils die unter den obigen Nummern E1 bis E7 aufgeführten Zitronensäureester und deren Mengen als Korrosionsinhibitor verwendet, d. h in jeder der kosmetischen Zusammensetzung K1 bis K60 werden jeweils die unter den Nummern E1 und E2 und E3 und E4 und E5 und E6 und E7 und E8 angeführten Zitronensäureester in den entsprechenden Mengen als Korrosionsinhibitor verwendet.

| | K1 | K2 | K3 | K4 |
|---|---|---|---|---|
| Elektrolyt | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Treibmittel | Ad 100 | Ad 100 | Ad 100 | Ad 100 |

| | K5 | K6 | K7 | K8 |
|---|---|---|---|---|
| Elektrolyt ¹⁾ | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Propan und/oder Butan | Ad 100 | Ad 100 | Ad 100 | Ad 100 |

| | K9 | K10 | K11 | K12 |
|---|---|---|---|---|
| Elektrolyt ¹⁾ | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Propan und/oder Butan (15:85) | Ad 100 | Ad 100 | Ad 100 | Ad 100 |

| | K13 | K14 | K15 | K16 |
|---|---|---|---|---|
| N-Alkylamidoalkyl-N,N-dialkyl-N-[hydroxyalkyl]ammonio]phosphaten der Formel (II) | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Propan und/oder Butan | Ad 100 | Ad 100 | Ad 100 | Ad 100 |

| | K17 | K18 | K19 | K20 |
|---|---|---|---|---|
| N-Octyl-1-[10-(4-octyliminopyridin-1-yl)decyl]pyridin-4-iminhydrochlorid | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Propan und/oder Butan | Ad 100 | Ad 100 | Ad 100 | Ad 100 |

| | K21 | K22 | K23 | K24 |
|---|---|---|---|---|
| 1-Hexadecyl-pyridiniumchloriden | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Propan und/oder Butan | Ad 100 | Ad 100 | Ad 100 | Ad 100 |

| | K25 | K26 | K27 | K28 |
|---|---|---|---|---|
| Silbercitraten | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Propan und/oder Butan | Ad 100 | Ad 100 | Ad 100 | Ad 100 |

| | K29 | K30 | K31 | K32 |
|---|---|---|---|---|
| Zink-bis[(9Z,12R)-12-hydroxy-9-octadecenoat] | 0,01 bis 1,0 | 0,01 bis 0,3 | 0,02 bis 0,2 | 0,03 bis 0,1 |
| Wasser | 0,02 bis 2,0 | 0,05 bis 1,8 | 0,2 bis 1,5 | 0,5 bis 1,5 |
| C₁-C₄-Alkohol | 15 bis 30 | 16 bis 29 | 18 bis 28 | 20 bis 24 |
| Propan und/oder Butan | Ad 100 | Ad 100 | Ad 100 | Ad 100 |

| | K33 | K34 | K35 | K36 |
|---|---|---|---|---|
| Elektrolyt | 0,01 bis 1,0 | 0,01 bis 0,3 | 0,02 bis 0,2 | 0,03 bis 0,1 |
| Ethanol | 15 bis 30 | 17 bis 29 | 18 bis 28 | 20 bis 24 |
| Wasser | 0,02 bis 2,0 | 0,05 bis 1,8 | 0,2 bis 1,5 | 0,5 bis 1,5 |
| Treibmittel | Ad 100 | Ad 100 | Ad 100 | Ad 100 |

| | K37 | K38 | K39 | K40 |
|---|---|---|---|---|
| Elektrolyt ¹⁾ | 0,01 bis 1,0 | 0,01 bis 0,3 | 0,02 bis 0,2 | 0,03 bis 0,1 |
| Ethanol | 15 bis 30 | 17 bis 29 | 18 bis 28 | 20 bis 24 |
| Wasser | 0,02 bis 2,0 | 0,05 bis 1,8 | 0,2 bis 1,5 | 0,5 bis 1,5 |
| Treibmittel | Ad 100 | Ad 100 | Ad 100 | Ad 100 |

| | K41 | K42 | K43 | K44 |
|---|---|---|---|---|
| N-Alkylamidoalkyl-N,N-dialkyl-N-[hyd roxyalkyl]ammonio]phosphaten der Formel (II) | 0,01 bis 1,0 | 0,01 bis 0,3 | 0,02 bis 0,2 | 0,03 bis 0,1 |
| Ethanol | 15 bis 30 | 17 bis 29 | 18 bis 28 | 20 bis 24 |
| Wasser | 0,02 bis 2,0 | 0,05 bis 1,8 | 0,2 bis 1,5 | 0,5 bis 1,5 |
| Propan und/oder Butan | Ad 100 | Ad 100 | Ad 100 | Ad 100 |

| | K45 | K46 | K47 | K48 |
|---|---|---|---|---|
| N-Octyl-1-[10-(4-octyliminopyridin-1-yl)decyl]pyridin-4-iminhydrochlorid | 0,01 bis 1,0 | 0,01 bis 0,3 | 0,02 bis 0,2 | 0,03 bis 0,1 |
| Ethanol | 15 bis 30 | 17 bis 29 | 18 bis 28 | 20 bis 24 |
| Wasser | 0,02 bis 2,0 | 0,05 bis 1,8 | 0,2 bis 1,5 | 0,5 bis 1,5 |
| Propan und/oder Butan | Ad 100 | Ad 100 | Ad 100 | Ad 100 |

| | K49 | K50 | K51 | K52 |
|---|---|---|---|---|
| 1-Hexadecyl-pyridiniumchloriden | 0,01 bis 1,0 | 0,01 bis 0,3 | 0,02 bis 0,2 | 0,03 bis 0,1 |
| Ethanol | 15 bis 30 | 17 bis 29 | 18 bis 28 | 20 bis 24 |
| Wasser | 0,02 bis 2,0 | 0,05 bis 1,8 | 0,2 bis 1,5 | 0,5 bis 1,5 |
| Propan und/oder Butan | Ad 100 | Ad 100 | Ad 100 | Ad 100 |

| | K53 | K54 | K55 | K56 |
|---|---|---|---|---|
| Silbercitraten | 0,01 bis 1,0 | 0,01 bis 0,3 | 0,02 bis 0,2 | 0,03 bis 0,1 |
| Ethanol | 15 bis 30 | 17 bis 29 | 18 bis 28 | 20 bis 24 |
| Wasser | 0,02 bis 2,0 | 0,05 bis 1,8 | 0,2 bis 1,5 | 0,5 bis 1,5 |
| Propan und/oder Butan | Ad 100 | Ad 100 | Ad 100 | Ad 100 |

| | K57 | K58 | K59 | K60 |
|---|---|---|---|---|
| Zink-bis[(9Z,12R)-12-hydroxy-9-octadecenoat] | 0,01 bis 1,0 | 0,01 bis 0,3 | 0,02 bis 0,2 | 0,03 bis 0,1 |
| Ethanol | 15 bis 30 | 17 bis 29 | 18 bis 28 | 20 bis 24 |
| Wasser | 0,02 bis 2,0 | 0,05 bis 1,8 | 0,2 bis 1,5 | 0,5 bis 1,5 |
| Propan und/oder Butan | Ad 100 | Ad 100 | Ad 100 | Ad 100 |

| | | | | |
|---|---|---|---|---|
| ¹⁾ Elektrolyt ist ausgewählt aus der Gruppe von N-Alkylamidoalkyl-N,N-dialkyl-N-[hydroxyalkyl]ammonio]phosphaten der obigen Formel (II), N-Octyl-1-[10-(4-octyliminopyridin-1-yl)decyl]pyridin-4-iminhydrochlorid, 1-Hexadecyl-pyridiniumchloriden, Silbercitraten, Zink-bis[(9Z,12R)-12-hydroxy-9-octadecenoat] sowie deren Mischungen | | | | |

Die erfindungsgemäße Verwendung des Zitronensäureesters der Formel (I) bzw. (la) in den zuvor genannten besonders bevorzugten Ausführungsformen führt zu einer hervorragenden Korrosionsinhibierung. Hierdurch wird ein Einsatz von wasserhaltigen alkoholischen Lösungsmitteln, insbesondere wasserhaltigem Ethanol, in Kombination mit als Elektrolyt vorliegenden Deodorantwirkstoffen in kosmetischen Zusammensetzungen ermöglicht, welche als Aerosol aus metallhaltigen Behältern versprühbar sind.

Ein zweiter Gegenstand der vorliegenden Erfindung ist ein Verfahren zur Korrosionsinhibierung von metallischen Aerosolbehältern und/oder Ventiltellern, wobei eine alkoholische Zusammensetzung, welche mindestens ein Treibmittel, mindestens einen Elektrolyten in einer Gesamtmenge von 0,01 bis 1,0 Gew.-% sowie Wasser in einer Gesamtmenge von 0,02 bis 2,0 Gew.-%, jeweils bezogen auf das Gesamtgewicht der alkoholischen kosmetischen Zusammensetzung, enthält mit einem Ester der Zitronensäure der Formel (I) 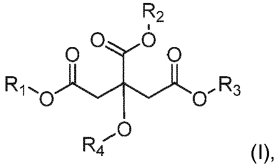 worin
R₁ bis R₃, jeweils unabhängig voneinander, für eine C₁-C₁₀-Alkylgruppe stehen, und
R₄ für Wasserstoff oder eine C₁-C₄-Alkylgruppe steht,
vermischt und in eine Aerosoldose gefüllt wird, welche anschließend mit mindestens einem Treibmittel beaufschlagt und mittels eines Ventils mit Ventilteller verschlossen wird.

Das erfindungsgemäße Verfahren resultiert gegenüber Verfahren, in welchen keine Ester der Zitronensäure der Formel (I) eingesetzt werden, in einer deutlich verbesserten Inhibierung der Korrosion von Aerosolbehältern und/oder Ventiltellern.

Die folgenden Beispiele erläutern die vorliegende Erfindung, ohne sie jedoch darauf einzuschränken:

### Beispiele:

### 1. Korrosionstest

Es wurden die folgenden kosmetischen Zusammensetzungen hergestellt, wobei als Zitronensäureester der Formel (I) bevorzugt Ester der Formel (la) eingesetzt wurden (alle Angaben in Gew.-%):

| Rohstoff | V | E* |
|---|---|---|
| Ethanol, 96 %ig (enthält 4 Gew.-% Wasser) | 23,7 | 22,2 |
| Zitronensäureester der Formel (I) | -- | 1,50 |
| Elektrolyt ¹⁾ | 0,075 | 0,075 |
| Konservierungsmittel ²⁾ | 0,125 | 0,125 |
| Parfum | 1,0 | 1,0 |
| Treibmittel ³⁾ | Ad 100 | Ad 100 |

| | | |
|---|---|---|
| 1) Arlasilk PTC (INCI-Bezeichnung: Cocamidopropyl PG-Dimonium Chlorid Phosphat; 45 - 48 Gew.-%ige Lösung in Wasser; Croda) 2) Symdiol 68T (INCI-Bezeichnung: 1,2-Hexanediol, Caprylyl Glycol, Tropolone; Symrise) 3) Propan/Butan im Verhältnis 15:85 * erfindungsgemäß | | |

Die Zusammensetzung V enthält 0,95 Gew.-% Wasser, welches aus dem Einsatz von wasserhaltigem Ethanol mit einem Wasseranteil von 4 Gew.-% stammt. Die Zusammensetzung E weist aufgrund des Einsatzes von wasserhaltigem Ethanol einen Wasseranteil von 0,89 Gew.-% auf.

Die kosmetischen Zusammensetzungen V (nicht erfindungsgemäß) und E (erfindungsgemäß) wurden in Aerosolsprühdosen aus Aluminium (Ball Aerocan) mit einer PPG7940 Epoxy-Innenbeschichtung befüllt. Als Ventil wurden Tellerdeckel aus unbeschichtetem Weißblech verwendet. Die befüllten Aerosoldosen wurden anschließend für 24 Wochen jeweils bei 23°C und 40 °C aufrecht sowie über Kopf stehend gelagert.

Bei den Aerosoldosen, welche die nicht erfindungsgemäße Zusammensetzung V enthalten, trat nach 4 Wochen bei 40 °C bzw. nach 8 Wochen bei 23 °C eine deutliche Korrosion der Ventilteller auf. Hingegen wurde bei den Aerosoldosen, welche mit der erfindungsgemäßen Zusammensetzung E befüllt waren, selbst nach 24 Wochen Lagerung bei 23°C und 40°C keine Korrosion der Ventilteller festgestellt. Der Zusatz des Zitronensäureesters der Formel (I), insbesondere der Formel (la), führt daher zu einem effektiven Korrosionsschutz von metallhaltigen Aerosolbehältern, welche mit wässrigen, elektrolythaltigen kosmetischen Zusammensetzungen befüllt sind.

### 3. Versprühbare Deodorantformulierungen:

Der in den nachfolgenden Beispielen eingesetzte Zitronensäureester der Formel (I) ist bevorzugt ein Zitronensäureester der Formel (la):

| | | | | | | |
|---|---|---|---|---|---|---|
| Rohstoff | 1 | 2 | 3 | 4 | 5 | 6 |
| Ethanol, 96%ig | 87,5 | 87,8 | 87,6 | 87,5 | 87,8 | 87,7 |
| Zitronensäureester der Formel (I) | 6,0 | 6,0 | 6,0 | 6,0 | 6,0 | 6,0 |
| Vitamin E Acetat | 0,2 | 0,2 | 0,2 | 0,2 | 0,2 | 0,2 |
| Arlasilk PTC | 0,3 | 0,3 | 0,3 | 0,3 | 0,3 | 0,3 |
| Sensiva SC 50 | 0,5 | 0,5 | 0,5 | 0,5 | -- | 0,3 |
| Symdiol 68 T | 0,5 | -- | 0,2 | -- | -- | -- |
| Tego Cosmo P 813 | -- | 0,25 | 0,25 | -- | -- | -- |
| SymDeo B125 | -- | -- | -- | 0,5 | 0,5 | -- |
| Dermosoft GMCY | -- | -- | -- | -- | 0,25 | -- |
| Dermosoft DGMC | -- | -- | -- | -- | -- | 0,2 |
| Melafresh T96-SC | -- | -- | -- | -- | -- | 0,1 |
| Phenoxyethanol | 1,0 | 1,0 | 1,0 | 1,0 | 1,0 | 1,0 |
| Parfum | 4,0 | 4,0 | 4,0 | 4,0 | 4,0 | 4,0 |

Die Rezepturen 1 bis 6 wurden im Gewichtsverhältnis 1 : 3 mit einer Mischung aus Propan und Butan (Verhältnis Propan/Butan = 15/85) in metallhaltige Aerosoldosen abgefüllt.

Es wurden die folgenden Handelsprodukte eingesetzt:

| Handelsprodukt | INCI | Lieferant/Hersteller |
|---|---|---|
| Arlasilk | Cocamidopropyl PG-Dimonium Chlorid Phosphat | Croda |
| Dermosoft GMCY | Glyceryl Caprylate | Dr. Straetmans |
| Dermosoft DGMC | Polyglyceryl-2 Caprate | Dr. Straetmans |
| Melafresh T96-SC | Melaleuca Alternifolia (Tea Tree) Leaf Oil | Southern Cross Botanicals P/L |
| Sensiva SC 50 | ETHYLHEXYLOXYGLYCERIN | Schülke & Mayr |
| SymDeo B125 | 2-Methyl 5-Cyclohexylpentanol | Symrise |
| Symdiol 68 T | 1,2-Hexanediol, Caprylyl Glycol, Tropolone | Symrise |
| Tego Cosmo P 813 | POLYGLYCERYL-3 CAPRYLATE | Evonik |

## Patentansprüche

1. Verwendung eines Esters der Zitronensäure der Formel (I) 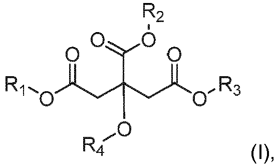 worin
R₁ bis R₃, jeweils unabhängig voneinander, für eine C₁-C₁₀-Alkylgruppe stehen, und
R₄ für Wasserstoff oder eine C₁-C₄-Alkylgruppe steht,
als Korrosionsinhibitor in alkoholischen kosmetischen Zusammensetzungen, welche mindestens ein Treibmittel, mindestens einen Elektrolyten in einer Gesamtmenge von 0,01 bis 1,0 Gew.-% sowie Wasser in einer Gesamtmenge von 0,02 bis 2,0 Gew.-%, jeweils bezogen auf das Gesamtgewicht der alkoholischen kosmetischen Zusammensetzung, enthalten.

2. Verwendung nach Anspruch 1, **dadurch gekennzeichnet, dass** in der Formel (I) die Reste R₁ bis R₃, jeweils unabhängig voneinander, für eine C₁-C₈-Alkylgruppe, vorzugsweise für eine C₁-C₆-Alkylgruppe, bevorzugt für eine C₁-C₄-Alkylgruppe, insbesondere für eine C₂-Alkylgruppe, stehen.

3. Verwendung nach einem der Ansprüche 1 oder 2, **dadurch gekennzeichnet, dass** in der Formel (I) der Rest R₄ für Wasserstoff steht.

4. Verwendung nach einem der vorangehenden Ansprüche , **dadurch gekennzeichnet, dass** ein Ester der Zitronensäure der Formel (la) 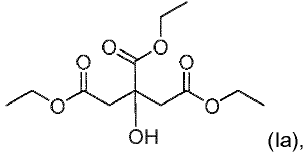 verwendet wird.

5. Verwendung nach einem der vorangehenden Ansprüche , **dadurch gekennzeichnet**, das der Ester der Zitronensäure der Formel (I) oder der Formel (la) in einer Gesamtmenge von 0,5 bis 5,0 Gew.-%, vorzugsweise von 0,5 bis 4,0 Gew.-%, bevorzugt von 0,5 bis 3,0 Gew.-%, insbesondere von 1,0 bis 2,0 Gew.-%, bezogen auf das Gesamtgewicht der alkoholischen kosmetischen Zusammensetzung, verwendet wird.

6. Verwendung nach einem der vorangehenden Ansprüche , **dadurch gekennzeichnet, dass** die alkoholische kosmetische Zusammensetzung mindestens einen einwertigen C₁-C₄-Alkohol, ausgewählt aus der Gruppe von Methanol, Ethanol, Propanol, iso-Propanol, Butanol sowie deren Mischungen, insbesondere Ethanol, in einer Gesamtmenge von 15 bis 30 Gew.-%, vorzugsweise 18 bis 28 Gew.-%, insbesondere 20 bis 24 Gew.-%, bezogen auf das Gesamtgewicht der alkoholischen kosmetischen Zusammensetzung, enthält.

7. Verwendung nach einem der vorangehenden Ansprüche , **dadurch gekennzeichnet, dass** die kosmetische Zusammensetzung Wasser in einer Gesamtmenge von 0,05 bis 1,8 Gew.-%, vorzugsweise von 0,1 bis 1,6 Gew.-%, bevorzugt von 0,2 bis 1,5 Gew.-%, insbesondere von 0,5 bis 1,5 Gew.-%, bezogen auf das Gesamtgewicht der alkoholischen kosmetischen Zusammensetzung, enthält.

8. Verwendung nach einem der vorangehenden Ansprüche , **dadurch gekennzeichnet, dass** der mindestens eine Elektrolyt ausgewählt ist aus der Gruppe von anorganischen Salzen, insbesondere Alkalimetalsalzen; Salzen von di- und trivalenten Kationen sowie N-Alkylamidoalkyl-N,N-dialkyl-N-[hydroxyalkyl]ammonio]phosphaten; organischen Salzen, insbesondere N-Octyl-1-[10-(4-octyliminopyridin-1-yl)decyl]pyridin-4-iminhydrochlorid, 1-Hexadecylpyridiniumchloriden, Silbercitraten, Zink-bis[(9Z,12R)-12-hydroxy-9-octadecenoat], Kaliumcapryloylglutamaten, Benzalkoniumchloriden, Benzethoniumchloriden, 2-Butyloctansäuren, Calciummagnesiumsilikaten, Hexadecyltrimethylammoniumchloriden; anorganischen und organischen Säuren, anorganischen und organischen Basen, insbesondere Ammoniak und Aminen, sowie deren Mischungen.

9. Verwendung nach Anspruch 7, **dadurch gekennzeichnet, dass** der mindestens eine Elektrolyt ausgewählt ist aus der Gruppe von N-Alkylamidoalkyl-N,N-dialkyl-N-[hydroxyalkyl]ammonio]phosphaten der Formel (II) 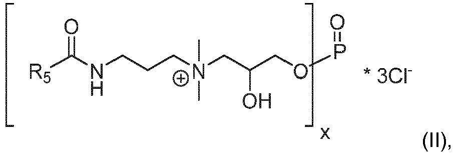 worin
R₅ für einen C₈-C₁₈-Alkylrest, insbesondere einen Cocoylrest, steht, N-Octyl-1-[10-(4-octyliminopyridin-1-yl)decyl]pyridin-4-iminhydrochlorid, 1-Hexadecyl-pyridiniumchloriden, Silbercitraten, Zink-bis[(9Z,12R)-12-hydroxy-9-octadecenoat] sowie deren Mischungen.

10. Verwendung nach einem der vorangehenden Ansprüche , **dadurch gekennzeichnet, dass** die kosmetische Zusammensetzung den mindestens einen Elektrolyten in einer Gesamtmenge von 0,01 bis 0,3 Gew.-%, vorzugsweise von 0,02 bis 0,2 Gew.-%, insbesondere von 0,03 bis 0,1 Gew.-%, bezogen auf das Gesamtgewicht der alkoholischen kosmetischen Zusammensetzung, enthält.

11. Verwendung nach einem der vorangehenden Ansprüche , **dadurch gekennzeichnet, dass** die kosmetische Zusammensetzung das mindestens eine Treibmittel in einer Gesamtmenge von 50 bis 95 Gew.%, vorzugsweise 60 bis 85 Gew.%, insbesondere 70 bis 80 Gew.-%, bezogen auf das Gesamtgewicht der alkoholischen kosmetischen Zusammensetzung, enthält.

12. Verwendung nach einem der vorangehenden Ansprüche , **dadurch gekennzeichnet, dass** mindestens ein Zitronensäureester der Formel (la) 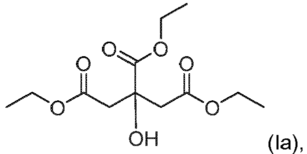 als Korrosionsinhibitor in alkoholischen kosmetischen Zusammensetzungen, welche Propan und/oder Butan in einer Gesamtmenge von 70 bis 80 Gew.-%, mindestens einen Elektrolyten in einer Gesamtmenge von 0,03 bis 0,1 Gew.-% sowie Wasser in einer Gesamtmenge von 0,5 bis 1,5 Gew.-%, jeweils bezogen auf das Gesamtgewicht der alkoholischen kosmetischen Zusammensetzung, enthalten, verwendet wird,
wobei der mindestens eine Elektrolyt ausgewählt ist aus der Gruppe von N-Alkylamidoalkyl-N,N-dialkyl-N-[hydroxyalkyl]ammonio]phosphaten der Formel (II) 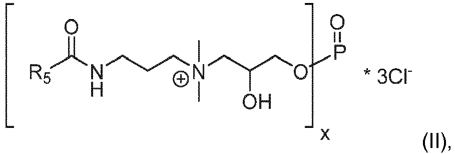 worin
R₅ für einen C₈-C₁₈-Alkylrest, insbesondere einen Cocoylrest, steht, N-Octyl-1-[10-(4-octyliminopyridin-1-yl)decyl]pyridin-4-iminhydrochlorid, 1-Hexadecyl-pyridiniumchloriden, Silbercitraten, Zink-bis[(9Z,12R)-12-hydroxy-9-octadecenoat] sowie deren Mischungen und
wobei der Zitronensäureester der Formel (la) in einer Gesamtmenge von 1,0 bis 2,0 Gew.-%, bezogen auf das Gesamtgewicht der alkoholischen kosmetischen Zusammensetzung, verwendet wird.

13. Verwendung nach einem der vorangehenden Ansprüche , **dadurch gekennzeichnet, dass** die kosmetische Zusammensetzung zusätzlich ein von Wasser verschiedenes Lösungsmittel, ausgewählt aus der Gruppe von Ethylenglykol, 1,2-Propylenglykol, 1,3-Propylenglykol, Glyzerin, n-Butanol, 1,3-Butylenglykol sowie deren Mischungen in einer Gesamtmenge von 15 bis 30 Gew.-%, vorzugsweise 18 bis 28 Gew.-%, insbesondere 20 bis 24 Gew.-%, bezogen auf das Gesamtgewicht der kosmetischen Zusammensetzung, enthält.

14. Verwendung nach einem der vorangehenden Ansprüche , **dadurch gekennzeichnet, dass** die alkoholischen kosmetische Zusammensetzung eine Deodorantzusammensetzung ist, welche als Aerosol versprühbar ist.

15. Verfahren zur Korrosionsinhibierung von metallischen Aerosolbehältern und/oder Ventiltellern, wobei eine alkoholische Zusammensetzung, welche mindestens ein Treibmittel, mindestens einen Elektrolyten in einer Gesamtmenge von 0,01 bis 1,0 Gew.-% sowie Wasser in einer Gesamtmenge von 0,02 bis 2,0 Gew.-%, jeweils bezogen auf das Gesamtgewicht der alkoholischen kosmetischen Zusammensetzung, enthält mit einem Ester der Zitronensäure der Formel (I) 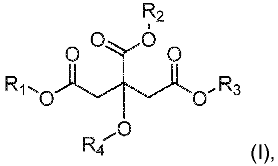 worin
R₁ bis R₃, jeweils unabhängig voneinander, für eine C₁-C₁₀-Alkylgruppe stehen, und
R₄ für Wasserstoff oder eine C₁-C₄-Alkylgruppe steht,
vermischt und in eine Aerosoldose gefüllt wird, welche anschließend mit mindestens einem Treibmittel beaufschlagt und mittels eines Ventils mit Ventilteller verschlossen wird

## Claims

1. The use of an ester of citric acid of formula (I) 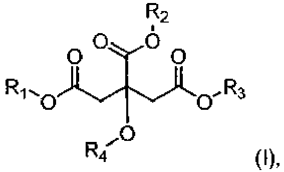 where
R₁ to R₃ represent, each independently of one another, a C₁-C₁₀ alkyl group, and
R₄ represents hydrogen or a C₁-C₄ alkyl group,
as a corrosion inhibitor in alcoholic cosmetic compositions which contain at least one propellant, at least one electrolyte in a total amount of from 0.01 to 1.0 wt.% and water in a total amount of from 0.02 to 2.0 wt.%, in each case based on the total weight of the alcoholic cosmetic composition.

2. The use according to claim 1, **characterized in that**, in formula (I), the functional groups R₁ to R₃ represent, each independently of one another, a C₁-C₈ alkyl group, preferably a C₁-C₆ alkyl group, more preferably a C₁-C₄ alkyl group, in particular a C₂ alkyl group.

3. The use according to one of claims 1 or 2, **characterized in that**, in formula (I), the functional group R₄ represents hydrogen.

4. The use according to one of the preceding claims, **characterized in that** an ester of citric acid of formula (Ia) 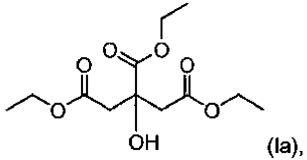 is used.

5. The use according to one of the preceding claims, **characterized in that** the ester of citric acid of formula (I) or formula (Ia) is used in a total amount of from 0.5 to 5.0 wt.%, more preferably of from 0.5 to 4.0 wt.%, preferably of from 0.5 to 3.0 wt.%, in particular of from 1.0 to 2.0 wt.%, based on the total weight of the alcoholic cosmetic composition.

6. The use according to one of the preceding claims, **characterized in that** the alcoholic cosmetic composition contains at least one monovalent C₁-C₄ alcohol, selected from the group of methanol, ethanol, propanol, iso-propanol, butanol and mixtures thereof, in particular ethanol, in a total amount of from 15 to 30 wt.%, preferably from 18 to 28 wt.%, in particular from 20 to 24 wt.%, based on the total weight of the alcoholic cosmetic composition.

7. The use according to one of the preceding claims, **characterized in that** the cosmetic composition contains water in a total amount of from 0.05 to 1.8 wt.%, preferably of from 0.1 to 1.6 wt.%, more preferably of from 0.2 to 1.5 wt.%, in particular of from 0.5 to 1.5 wt.%, based on the total weight of the alcoholic cosmetic composition.

8. The use according to one of the preceding claims, **characterized in that** the at least one electrolyte is selected from the group of inorganic salts, in particular alkali metal salts; salts of divalent and trivalent cations and N-alkylamidoalkyl-N,N-dialkyl-N-[hydroxyalkyl]ammonio] phosphates; organic salts, in particular N-octyl-1-[10-(4-octyliminopyridin-1-yl)decyl]pyridin-4-imine hydrochloride, 1-hexadecylpyridinium chlorides, silver citrates, zinc-bis[(9Z,12R)-12-hydroxy-9-octadecenoate], potassium capryloyl glutamates, benzalkonium chlorides, benzethonium chlorides, 2-butyloctanoic acids, calcium magnesium silicates, hexadecyltrimethylammonium chlorides; inorganic and organic acids, inorganic and organic bases, in particular ammonia and amines, and mixtures thereof.

9. The use according to claim 7, **characterized in that** the at least one electrolyte is selected from the group of N-alkylamidoalkyl-N,N-dialkyl-N-[hydroxyalkyl]ammonio] phosphates of formula (II) 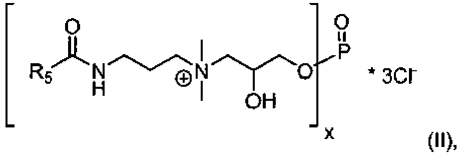 where
R₅ represents a C₈-C₁₈ alkyl functional group, in particular a cocoyl functional group, N-octyl-1-[10-(4-octyliminopyridin-1-yl)decyl]pyridin-4-imine hydrochloride, 1-hexadecylpyridinium chlorides, silver citrates, zinc-bis[(9Z,12R)-12-hydroxy-9-octadecenoate] and mixtures thereof.

10. The use according to one of the preceding claims, **characterized in that** the cosmetic composition contains the at least one electrolyte in a total amount of from 0.01 to 0.3 wt.%, preferably of from 0.02 to 0.2 wt.%, in particular of from 0.03 to 0.1 wt.%, based on the total weight of the alcoholic cosmetic composition.

11. The use according to one of the preceding claims, **characterized in that** the cosmetic composition contains the at least one propellant in a total amount of from 50 to 95 wt.%, preferably from 60 to 85 wt.%, in particular from 70 to 80 wt.%, based on the total weight of the alcoholic cosmetic composition.

12. The use according to one of the preceding claims, **characterized in that** at least one citric acid ester of formula (Ia) 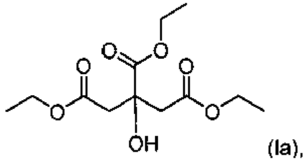 is used as a corrosion inhibitor in alcoholic cosmetic compositions which contain propane and/or butane in a total amount of from 70 to 80 wt.%, at least one electrolyte in a total amount of from 0.03 to 0.1 wt.%, and water in a total amount of from 0.5 to 1.5 wt.%, in each case based on the total weight of the alcoholic cosmetic composition, the at least one electrolyte being selected from the group of N-alkylamidoalkyl-N,N-dialkyl-N-[hydroxyalkyl]ammonio] phosphates of formula (II) 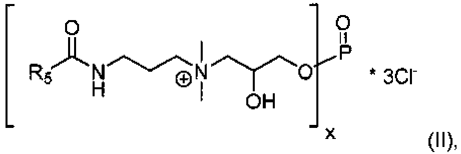 where
R₅ represents a C₈-C₁₈ alkyl radical, in particular a cocoyl functional group, N-octyl-1-[10-(4-octyliminopyridin-1-yl)decyl]pyridin-4-imine hydrochloride, 1-hexadecylpyridinium chlorides, silver citrates, zinc-bis[(9Z,12R)-12-hydroxy-9-octadecenoate] and mixtures thereof and the citric acid ester of formula (Ia) being used in a total amount of from 1.0 to 2.0 wt.%, based on the total weight of the alcoholic cosmetic composition.

13. The use according to one of the preceding claims, **characterized in that** the cosmetic composition additionally contains a solvent other than water, selected from the group of ethylene glycol, 1,2-propylene glycol, 1,3-propylene glycol, glycerol, n-butanol, 1,3-butylene glycol and mixtures thereof in a total amount of from 15 to 30 wt.%, preferably from 18 to 28 wt.%, in particular from 20 to 24 wt.%, based on the total weight of the cosmetic composition.

14. The use according to one of the preceding claims, **characterized in that** the alcoholic cosmetic composition is a deodorant composition which can be sprayed as an aerosol.

15. A method for inhibiting corrosion of metal aerosol containers and/or valve discs, wherein an alcoholic composition which contains at least one propellant, at least one electrolyte in a total amount of from 0.01 to 1.0 wt.% and water in a total amount of from 0.02 to 2.0 wt.%, in each case based on the total weight of the alcoholic cosmetic composition, is mixed with an ester of citric acid of formula (I) 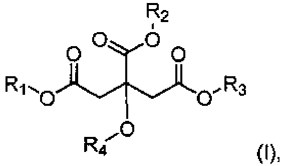 where
R₁ to R₃ represent, each independently of one another, a C₁-C₁₀ alkyl group, and R₄ represents hydrogen or a C₁-C₄ alkyl group,
and filled into an aerosol can, which is then acted upon by at least one propellant and sealed by means of a valve having valve discs.

## Revendications

1. Utilisation d'un ester d'acide citrique de formule (I) 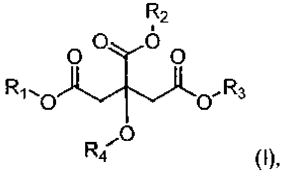 dans laquelle
R₁ à R₃, chacun indépendamment des autres, représentent un groupe alkyle en C₁-C₁₀, et
R₄ représente l'hydrogène ou un groupe alkyle en C₁-C₄,
comme inhibiteur de corrosion dans des composition cosmétiques alcooliques comprenant au moins un agent propulseur, au moins un électrolyte en une quantité totale de 0,01 à 1,0 % en poids et de l'eau en une quantité totale de 0,02 à 2,0 % en poids, dans chaque cas par rapport au poids total de la composition cosmétique alcoolique.

2. Utilisation selon la revendication 1, **caractérisée en ce que**, dans la formule (I), les radicaux R₁ à R₃, chacun indépendamment des autres, représentent un groupe alkyle en C₁-C₈, de préférence un groupe alkyle en C₁-C₆, de manière davantage préférée un groupe alkyle en C₁-C₄, en particulier un groupe alkyle en C₂.

3. Utilisation selon l'une quelconque des revendications 1 ou 2, **caractérisée en ce que**, dans la formule (I), le radical R₄ représente l'hydrogène.

4. Utilisation selon l'une quelconque des revendications précédentes, **caractérisée en ce qu'**un ester d'acide citrique de formule (Ia) 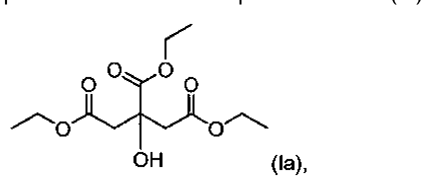 est utilisé.

5. Composition cosmétique selon l'une quelconque des revendications précédentes, **caractérisée en ce que** l'ester d'acide citrique de formule (I) ou de formule (la) est utilisé en une quantité totale de 0,5 à 5,0 % en poids, de préférence de 0,5 à 4,0 % en poids, de manière préférée de 0,5 à 3,0 % en poids, en particulier de 1,0 à 2,0 % en poids par rapport au poids total de la composition cosmétique alcoolique.

6. Utilisation selon l'une quelconque des revendications précédentes, **caractérisée en ce que** la composition cosmétique alcoolique contient au moins un monoalcool en C₁-C₄ sélectionné dans le groupe du méthanol, de l'éthanol, du propanol, de l'isopropanol, du butanol et de leurs mélanges, notamment l'éthanol, en une quantité totale de 15 à 30 % en poids, de préférence de 18 à 28 % en poids, en particulier de 20 à 24 % en poids, par rapport au poids total de la composition cosmétique alcoolique.

7. Utilisation selon l'une quelconque des revendications précédentes, **caractérisée en ce que** la composition cosmétique contient de l'eau en une quantité totale de 0,05 à 1,8 % en poids, de préférence de 0,1 à 1,6 % en poids, de manière préférée de 0,2 à 1,5 % en poids, en particulier de 0,5 à 1,5 % en poids, par rapport au poids total de la composition cosmétique alcoolique.

8. Utilisation selon l'une quelconque des revendications précédentes, **caractérisée en ce que** l'au moins un électrolyte est sélectionné dans le groupe des sels inorganiques, en particulier les sels de métaux alcalins ; des sels de cations di- et trivalents et des N-alkylamidoalkyl-N,N-dialkyl-N-[hydroxyalkyl]ammonio]phosphates ; des sels organiques, en particulier le chlorhydrate de N-octyl-1-[10-(4-octyliminopyridin-1-yl)décyl]pyridin-4-imine, les chlorures de 1-hexadécylpyridinium, les citrates d'argent, le bis[(9Z,12R)-12-hydroxy-9-octadécénoate] de zinc, des glutamates de capryloyle de potassium, des chlorures de benzalkonium, des chlorures de benzéthonium, des acides 2-butyloctanoïques, des silicates de calcium et de magnésium, des chlorures d'hexadécyltriméthylammonium ; des acides inorganiques et organiques, des bases inorganiques et organiques, en particulier l'ammoniac et les amines, et de leurs mélanges.

9. Utilisation selon la revendication 7, **caractérisée en ce que** l'au moins un électrolyte est choisi dans le groupe des N-alkylamidoalkyl-N,N-dialkyl-N-[hydroxyalkyl]ammonio]phosphates de formule (II) 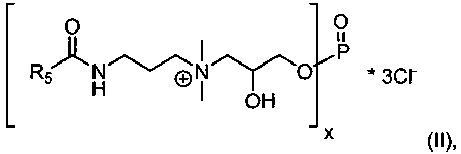 dans laquelle
R₅ représente un radical alkyle en C₈-C₁₈, en particulier un radical cocoyle, le chlorhydrate de N-octyl-1-[10-(4-octyliminopyridin-1-yl)décyl]pyridin-4-imine, les chlorures de 1-hexadécyl-pyridinium, les citrates d'argent, le bis[(9Z,12R)-12-hydroxy-9-octadécénoate] de zinc et leurs mélanges.

10. Utilisation selon l'une quelconque des revendications précédentes, **caractérisée en ce que** la composition cosmétique contient au moins un électrolyte en une quantité totale de 0,01 à 0,3 % en poids, de préférence de 0,02 à 0,2 % en poids, en particulier de 0,03 à 0,1 % en poids, par rapport au poids total de la composition cosmétique alcoolique.

11. Utilisation selon l'une quelconque des revendications précédentes, **caractérisée en ce que** la composition cosmétique contient au moins un agent propulseur en une quantité totale de 50 à 95 % en poids, de préférence de 60 à 85 % en poids, en particulier de 70 à 80 % en poids, par rapport au poids total de la composition cosmétique alcoolique.

12. Utilisation selon l'une quelconque des revendications précédentes, **caractérisée en ce que** l'on utilise au moins un ester d'acide citrique de formule (la) 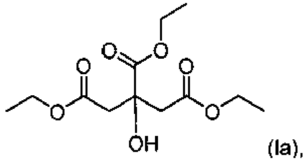 comme inhibiteur de corrosion dans des compositions cosmétiques alcooliques contenant du propane et/ou du butane en une quantité totale de 70 à 80 % en poids, au moins un électrolyte en une quantité totale de 0,03 à 0,1 % en poids et de l'eau en une quantité totale de 0,5 à 1,5 % en poids, dans chaque cas par rapport au poids total de la composition cosmétique alcoolique, l'au moins un électrolyte étant choisi dans le groupe des N-alkylamidoalkyl-N,N-dialkyl-N-[hydroxyalkyl]ammonio]phosphates de formule (II) 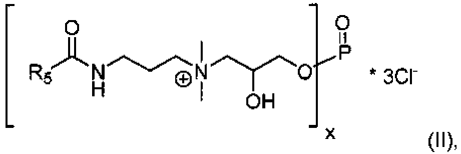 dans laquelle
R₅ représente un radical alkyle en C₈-C₁₈, en particulier un radical cocoyle, le chlorhydrate de N-octyl-1-[10-(4-octyliminopyridin-1-yl)décyl]pyridin-4-imine, les chlorures de 1-hexadécyl-pyridinium, les citrates d'argent, le bis[(9Z,12R)-12-hydroxy-9-octadécénoate] de zinc et leurs mélanges, et dans laquelle l'ester d'acide citrique de formule (la) est utilisé en une quantité totale de 1,0 à 2,0 % en poids par rapport au poids total de la composition cosmétique alcoolique.

13. Utilisation selon l'une quelconque des revendications précédentes, **caractérisée en ce que** la composition cosmétique contient en outre un solvant autre que l'eau, choisi dans le groupe de l'éthylèneglycol, du 1,2-propylèneglycol, du 1,3-propylèneglycol, du glycérol, du n-butanol, du 1,3-butylèneglycol et de leurs mélanges, en une quantité totale de 15 à 30 % en poids, de préférence 18 à 28 % en poids, en particulier de 20 à 24 % en poids, par rapport au poids total de la composition cosmétique.

14. Utilisation selon l'une quelconque des revendications précédentes, **caractérisée en ce que** la composition cosmétique alcoolique est une composition déodorante qui peut être pulvérisée sous forme d'aérosol.

15. Procédé pour inhiber la corrosion de récipients aérosols et/ou de corps de valve métalliques, dans lequel une composition alcoolique comprenant au moins un agent propulseur, au moins un électrolyte en une quantité totale de 0,01 à 1,0 % en poids et de l'eau en une quantité totale de 0,02 à 2,0 % en poids, dans chaque cas par rapport au poids total de la composition cosmétique alcoolique, est mélangé avec un ester d'acide citrique de formule (I) 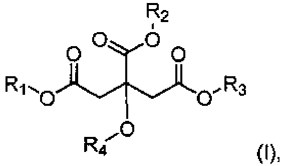 dans laquelle
R₁ à R₃, chacun indépendamment des uns des autres, représentent un groupe alkyle en C₁-C₁₀, et R₄ représente l'hydrogène ou un groupe alkyle en C₁-C₄,
et introduit dans un récipient d'aérosol, qui est ensuite soumis à l'action d'au moins un agent propulseur et fermé au moyen d'une soupape avec disque de soupape.
